# EUROPÄISCHE PATENTSCHRIFT

(11) **EP 3 838 205 B1**
(45) Veröffentlichungstag und Bekanntmachung des Hinweises auf die Patenterteilung: **05.11.2025**
(21) Anmeldenummer: 20212480.6
(22) Anmeldetag: 08.12.2020
(51) Int. Cl.: A61B 18/14, A61B 18/00

(54) **RESEKTOSKOP MIT DISTALER ELEKTRODENFÜHRUNG**
RESECTOSCOPE WITH DISTAL ELECTRODE GUIDE
RÉSECTOSCOPE À GUIDAGE DISTALE D'ÉLECTRODE

(30) Priorität: 20.12.2019 DE 102019135571
(43) Veröffentlichungstag der Anmeldung: 23.06.2021
(73) Patentinhaber: Olympus Winter & Ibe GmbH, 22045 Hamburg (DE)
(72) Erfinder: Brockmann, Christian, 21279 Hollenstedt (DE); Miersch, Hannes, 22149 Hamburg (DE)
(74) Vertreter: Hoener, Matthias

(56) Entgegenhaltungen:
- DE-A1- 102017 115 377
- DE-B3- 102017 118 885
- US-A- 3 752 159

## Beschreibung

Die Erfindung betrifft ein Elektrodensystem gemäß Anspruch 1 und ein Resektoskop für die endoskopische Chirurgie der im Oberbegriff des Anspruches 3 genannten Art.

Resektoskope der gattungsgemäßen Art werden vor allem in der Urologie bei chirurgischen Arbeiten in der Blase und in der Urethra verwendet. Sie werden üblicherweise zur Resektion und Vaporisation von Gewebe, zum Beispiel von Gewebe im unteren Harntrakt, verwendet. Dazu umfassen die Resektoskope ein längsverschiebbares elektrochirurgisches Elektrodeninstrument, das nach dem Einführen des Resektoskops mit seinem distalen Arbeitsende aus dem distalen Ende des Schaftrohres des Resektoskops hervorgeschoben werden kann. Darüber hinaus enthalten die Resektoskope zur Beobachtung der Eingriffsstelle und Überwachung des Eingriffs in der Regel ein optisches System, das am distalen Ende ein Objektiv umfasst und am proximalen Ende mit einem Okular zur direkten Beobachtung oder mit einer elektronischen Überwachungseinheit verbunden ist. Schließlich enthalten die Resektoskope zur Beleuchtung der Eingriffsstelle ein Beleuchtungselement, meist in Form eines Lichtleitfaserbündels, das den Schaft des Resektoskops durchspannt und an seinem proximalen Ende mit einer Lichtquelle verbunden ist.

Das durch den Resektoskopschaft hindurchgeführte Elektrodeninstrument kann an seinem distalen Arbeitsende eine elektrochirurgische Elektrode in Form einer Schlinge (Loop) oder eines Vaporisationsknopfes (PlasmaButton) umfassen. Solche Instrumente sind zum Beispiel die OES PRO Resektoskope (Olympus), die in der DE 10 2019 102 841 A1 und der US 4,917,082 A beschriebenen Resektoskope. Die Elektrodeninstrumente können als bipolare oder monopolare Instrumente ausgebildet sein, wobei bipolare Instrumente gegenüber monopolaren Instrumenten erhebliche Sicherheitsvorteile aufweisen können.

Die Elektrodeninstrumente sind in dem Instrumentenschaft längsverschiebbar, um das Abtragen von Gewebe mittels der distalen Elektrode zu ermöglichen, siehe auch DE 10 2017 115377 A1. Um eine gerade Führung der Elektrodeninstrumente durch den Instrumentenschaft zu gewährleisten, weisen die Elektrodeninstrumente an ihrem langgestreckten Schaftabschnitt in der Regel Führungselemente auf, die das Elektrodeninstrument an der Oberfläche der Optik oder an einer inneren Wand des Resektoskopschaftes abstützen. Dazu sind die Führungselemente üblicherweise komplementär zur Optik bzw. der inneren Wand teilkreisförmig ausgebildet, zum Beispiel in Form von teilkreisförmigen Führungsblechen.

Da die üblichen Elektrodeninstrumente einen relativ langen Hub von beispielsweise 23 mm haben, sind die Führungselemente in der Regel ebenso weit von dem distalen Ende des Elektrodeninstruments beabstandet. Auf diese Weise wird sichergestellt, dass die Führungselemente auch dann innerhalb des Resektoskopschaftes verbleiben, wenn das Elektrodeninstrument maximal weit nach distal verschoben ist. Dieser weite Abstand zwischen den Führungselementen und der distalen Elektrode hat aber zur Folge, dass das Elektrodeninstrument in diesem Bereich biegbar ist und sich die Elektrode gegenüber dem Resektoskopschaft daher verschieben oder sogar verbiegen kann. Darüber hinaus ist die Positionierung von distal bereits verbogenen Elektrodeninstrumenten nicht korrigierbar.

Da das distale Ende des Resektoskopschaftes häufig bei chirurgischen Eingriffen als Abschneidkante für Gewebe genutzt wird, ist es jedoch wichtig, dass die Elektrodenposition exakt zur Kante des distalen Resektoskopschaft-Endes führbar ist. Nur so kann ein gutes Schneidergebnis erzielt werden. Bereits kleinere Ungenauigkeiten in der Geradheit der Elektrodeninstrumente können negative Auswirkungen auf die Schneideigenschaften der Elektrodeninstrumente haben. Sitzt eine Schlingenelektrode zu tief, kann ein direkter Kontakt zwischen der Elektrode und der Schaftspitze zu einer Beschädigung der Elektrode führen. So kann die Schlinge beispielsweise beim Herausziehen an der Schaftspitze bzw. einem Isolieransatz hängen bleiben und verbiegen oder sogar brechen. Außerdem ist es denkbar, dass es zu Materialbeschädigungen am Schaft kommt. Sitzt eine Schlingenelektrode zu hoch, kann der Resektoskopschaft als Abschnittkante wegfallen und ein Abschneiden von Gewebe wird wenigstens erschwert.

Aufgabe der vorliegenden Erfindung ist es daher, ein Resektoskop bereitzustellen, dessen Elektrodeninstrumente in ihrem distalen Endbereich stärker stabilisiert sind.

### Beschreibung

Gelöst wird diese Aufgabe durch ein Elektrodensystem mit den Merkmalen von Anspruch 1 sowie durch ein Resektoskop mit den Merkmalen von Anspruch 3.

Die Erfindung betrifft insbesondere in einem ersten Aspekt ein Resektoskop für die endoskopische Chirurgie mit einem rohrartigen Schaft und einem Handgriff, wobei der Schaft ein längsverschieblich angeordnetes Elektrodeninstrument und an seinem distalen Ende einen elektrisch isolierenden Isoliereinsatz umfasst, wobei das Elektrodeninstrument einen langgestreckten Schaftabschnitt mit einem oder zwei Tragarmen und in seinem distalen Endbereich eine mit Hochfrequenzstrom beaufschlagbare Elektrode aufweist, dadurch gekennzeichnet, dass der Isoliereinsatz ein oder mehrere Führungselemente zur Halterung jeweils eines Tragarms aufweist, wobei der Tragarm in dem jeweiligen Führungselement längsverschiebbar ist.

Die erfindungsgemäße Anordnung der stabilisierenden Führungselemente in dem Isoliereinsatz an dem distalen Ende des Resektoskopschaftes minimiert den Abstand zwischen der Elektrodenspitze und den Führungselementen, wodurch eine sicherere Positionierung der Elektrode ermöglicht wird. Bei einer Längsverschiebung des Elektrodeninstruments werden die Führungselemente nicht mehr mitbewegt. Das Elektrodensystem wird hierdurch insgesamt deutlich stabiler und gegen Verbiegungen geschützt. Darüber hinaus sind auch präzisere Schnitte mit leicht verbogenen Elektrodeninstrumenten möglich, da die Elektroden beim Schneiden durch die Führungselemente in die korrekte Position zur Schneidkante des Schaftendes geführt werden. Das Schneidergebnis wird somit insgesamt deutlich verbessert.

Das erfindungsgemäße Resektoskop weist in üblicher Ausbildungsweise einen rohrartigen Schaft auf. Der Endoskopschaft umfasst ein langgestrecktes Hüllrohr. Neben dem Schaftteil umfasst das Resektoskop zum Halten und Bedienen einen Handgriff, der üblicherweise aus zwei Griffteilen besteht.

Der Schaft des Resektoskops weist ein längsverschieblich angeordnetes Elektrodeninstrument auf. Das Elektrodeninstrument wird als Durchgangsinstrument in einem solchen Resektoskop verwendet und kann zur einmaligen oder mehrmaligen Benutzung ausgebildet sein.

Das Elektrodeninstrument weist einen langgestreckten Schaftabschnitt (Schaftteil) auf und ist als Durchgangsinstrument für ein Resektoskop ausgebildet, d.h. als Instrument, das durch ein resektoskopisches Schaftrohr in eine Körperöffnung einführbar ist. Das Elektrodeninstrument weist an seinem distalen Ende eine mit Hochfrequenzstrom beaufschlagbare Elektrode auf. Bei der Elektrode kann es sich um eine Schneidschlinge, einen Vaporisationsknopf (PlasmaButton) oder andere handelsübliche Elektroden handeln. Bevorzugt ist die Elektrode eine Schneidschlingenelektrode. Entsprechende Elektroden und Elektrodeninstrumente sind Fachleuten bekannt.

Das Elektrodeninstrument kann ein bipolares Elektrodeninstrument sein, das die Elektrode als Teil einer Elektrodenanordnung umfasst. In diesem Falle wird das Elektrodeninstrument zum Beispiel eine zweite Elektrode im distalen Endbereich des Elektrodeninstruments umfassen, die als Neutralelektrode ausgebildet ist. Alternativ kann die zweite Elektrode (Neutralelektrode) auch an anderen Elementen des distalen Endbereichs des Resektoskops angeordnet sein. Selbstverständlich kann das Elektrodeninstrument auch als monopolares Instrument ausgebildet sein.

Das Elektrodeninstrument ist innerhalb des Schaftes eines Resektoskops längsverschiebbar, d. h. in axialer Richtung nach distal und proximal beweglich. Zum Anschluss an das Resektoskop weist das Elektrodeninstrument den langgestreckten Schaft auf, der zur Herstellung einer bewegungsgekoppelten Verbindung an seinem proximalen Ende an einem von dem Resektoskop umfassten Schlitten befestigbar ist. Der Schlitten gleitet typischerweise auf einem Rohr und wird über eine Federeinheit federvorgespannt in einer Ruhestellung gehalten. So kann die Elektrode am distalen Ende auf zu schneidendes Gewebe zu- oder von diesem wegbewegt werden, ohne das gesamte Resektoskop bewegen zu müssen. Darüber hinaus ist es durch die Längsverschiebbarkeit des Elektrodeninstruments möglich, zwischen der Elektrode und dem Isoliereinsatz Gewebe einzuklemmen und von der Eingriffsstelle zu entfernen. Das distale Ende des Resektoskopschaftes bzw. des Isoliereinsatzes und die Elektrode sind somit mittels der Längsverschiebbarkeit des Elektrodeninstruments aufeinander zu und voneinander weg beweglich.

Das Elektrodeninstrument weist ein oder zwei langgestreckte Tragarme auf, die den Schaftabschnitt des Elektrodeninstruments bilden. Elektrodeninstrumente mit zwei Tragarmen sind erfindungsgemäß bevorzugt. Weist das Elektrodeninstrument nur einen Tragarm auf, so ist die Elektrode an dessen distalen Ende angeordnet. Weist das Elektrodeninstrument zwei Tragarme auf, so sind diese an ihrem distalen Ende durch ein Verbindungselement miteinander verbunden. Das Verbindungselement kann beispielsweise die Elektrode ausbilden oder die Elektrode kann auf dem Verbindungselement angeordnet sein. Mit anderen Worten tragen die Tragarme die Elektrode oder ein Verbindungselement, das die Elektrode trägt. Der oder die Tragarme sind vorzugsweise langgestreckt. Weist das Elektrodeninstrument zwei Tragarme auf, so verlaufen diese im Wesentlichen parallel zueinander. Dabei können die beiden Tragarme beispielsweise entlang der Innenwand eines Innenrohres oder Hüllrohres des Resektoskops verlaufen und entlang des Innenumfangs dieser Innenwand durch etwa 120° bis 200° voneinander beabstandet sein, vorzugsweise etwa 180°. Es ist somit bevorzugt, dass die beiden Tragarme sich in etwa gegenüberliegen, wobei die Längsachse des Resektoskopschaftes den Mittelpunkt zwischen den beiden Tragarmen darstellt.

Neben dem Elektrodeninstrument weist der Schaft des Resektoskops an seinem distalen Ende einen Isoliereinsatz auf. Der Isoliereinsatz ist elektrisch nicht leitfähig, d.h. elektrisch isolierend, ausgebildet. Auf diese Weise wird die Isolierung der Aktivelektrode gegenüber dem leitfähigen Resektoskopschaft gewährleistet. Zu diesem Zweck ist der Isoliereinsatz vorzugsweise vollständig, mindestens aber in einem Maße, das die Isolierfähigkeit des Einsatzes gewährleistet, aus einem elektrisch nicht leitfähigen, d.h. elektrisch isolierenden, Material ausgebildet. Solche Materialien sind Fachleuten bekannt und umfassen beispielsweise Keramiken und Kunststoffe. Isoliereinsätze aus Kunststoffen sind erfindungsgemäß aufgrund der relativ geringen Produktionskosten und guten Isolierfähigkeit besonders bevorzugt. Da der Isoliereinsatz bei Verwendung während einer elektrochirurgischen Behandlung mit einer Elektrode in Kontakt mit dem entstehenden Plasma kommen kann, sind thermostabile Kunststoffe besonders bevorzugt. Thermostabile Kunststoffe sind in der Lage, die im Bereich der distalen Resektoskopspitze entstehenden hohen Temperaturen unbeschadet zu überstehen. Geeignete thermostabile Kunststoffe können beispielsweise ausgewählt sein aus der Gruppe bestehend aus Fluorpolymeren und Cycloolefin-Copolymeren. Die aus Kunststoff gefertigten Isoliereinsätze können mittels eines Spritzgussverfahrens gefertigt sein.

Der Isoliereinsatz ist zur lösbaren Verbindung mit dem distalen Endbereich eines Resektoskopschaftes geeignet. Dies bedeutet, dass Isoliereinsatz und der Endbereich mindestens abschnittsweise form- und größenkomplementär sind. Hierdurch ist der Isoliereinsatz mit dem Endbereich verbindbar. Jedenfalls ist die Verbindung derart fest bzw. derart gesichert, dass ein Ablösen des Isoliereinsatzes während eines operativen Eingriffs verhindert ist. So kann der Isoliereinsatz, der einen zylindrischen Abschnitt aufweist, beispielsweise auf das Innen- oder Hüllrohr aufgeschoben, in das Innenrohr eingeschoben oder zwischen Innen- und Hüllrohr eingeschoben werden.

Der Isoliereinsatz weist einen hohlen Abschnitt mit einem länglichen Hohlraum zum Hindurchführen von Durchgangsinstrumenten auf. Dieser Abschnitt ist im proximalen Endbereich des Isoliereinsatzes angeordnet. Er stellt sicher, dass durch den Resektoskopschaft geführte Durchgangsinstrumente durch das kanalförmige Innere des Isoliereinsatzes hindurchgeführt werden können. Der Isoliereinsatz kann eine im Wesentlichen zylindrische Form aufweisen oder mindestens einen im Wesentlichen zylindrischen proximalen Abschnitt. Entsprechend kann der Hohlraum im Inneren des hohlen Abschnitts eine Hohlzylinderform aufweisen.

Der erfindungsgemäße Isoliereinsatz weist ein oder mehrere Führungselemente zur Halterung eines Tragarms auf, d.h. 1, 2 oder mehr Führungselemente. Isoliereinsätze mit zwei Führungselementen sind erfindungsgemäß bevorzugt. Jedes Führungselement ist mit anderen Worten zur Halterung eines Tragarms ausgebildet. Weist das Elektrodeninstrument zwei Tragarme auf, so wird auch der Isoliereinsatz zwei entsprechende Führungselemente umfassen. Weist das Elektrodeninstrument dagegen nur einen Tragarm auf, so umfasst der Isoliereinsatz nur ein entsprechendes Führungselement.

Innerhalb des Führungselements ist der dem Führungselement zugeordnete Tragarm längsverschiebbar gelagert, d.h. derart gehalten, dass eine Verschiebung des Tragarms und damit auch des Elektrodeninstruments in Längsrichtung des Resektoskopschaftes möglich ist. Gleichzeitig verhindert das Führungselement eine Verschiebung des Tragarms in andere Richtungen, d.h. in Richtungen, die von der Längsrichtung des Resektoskopschaftes abweichen, zum Beispiel quer zur Längsrichtung (radial).

Um das Elektrodeninstrument zu stabilisieren, sind das oder die Führungselemente möglichst dicht am distalen Schaftende bzw. Ende des Isoliereinsatzes angeordnet. Der Abstand zwischen dem distalen Ende des Schafts und dem oder den Führungselementen kann beispielsweise 1,5 cm oder weniger, 1 cm oder weniger, 0,5 cm oder weniger, 0,25 cm oder weniger betragen. Vorzugsweise beträgt der Abstand 0,5 cm oder weniger.

Das oder die Führungselemente sind an der Innenwand des Isoliereinsatzes angeordnet. Dabei sind die Führungselemente so angeordnet und ausgebildet, dass sie die Blickachse der Optik nicht oder nur unwesentlich behindern. Die Führungselemente werden in der Regel in etwa parallel zur Längsachse (LS) des Resektoskopschaftes ausgebildet sein, d.h. die Längsachsen (LF) des oder der Führungselemente sind jeweils in etwa parallel zur Längsachse (LS) des Schaftes. Gleichzeitig erstrecken sich die Führungselemente von der Innenwand des Isoliereinsatzes in den Innenraum des Isoliereinsatzes, vorzugsweise in einem Winkel von 140° bis 40°, vorzugsweise in einem Winkel von 110° bis 70°, stärker bevorzugt in einem Winkel von etwa 90°, im Verhältnis zur Innenwand des Isoliereinsatzes. Dabei erstrecken sich die Führungselemente vorzugsweise nicht in einen Bereich des Innenraumes, der sich distal von der Optik oder distal von dem distalen Ende eines der Tragarme befindet.

Die Führungselemente können aus demselben isolierenden Material gefertigt sein, wie der Isoliereinsatz, d.h. beispielsweise aus einer Keramik. Alternativ sind aber auch Führungselemente aus den oben beschriebenen thermostabilen Kunststoffen denkbar. Es ist bevorzugt, dass die Führungselemente mit dem Rest des Isoliereinsatzes einteilig ausgebildet sind.

Das oder die Führungselemente umschließen dabei, wie an anderer Stelle hierin bereits erläutert, den jeweiligen Tragarm derart, dass eine Längsverschiebbarkeit des Tragarms gewährleistet ist und gleichzeitig ein Verschieben des Tragarms in andere Richtungen, z. B. quer zur Längsrichtung des Resektoskopschaftes, verhindert wird. Dies kann erreicht werden, indem das Führungselement den jeweiligen Tragarm auf 90° oder mehr seines Umfangs umschließt, vorzugsweise auf 180° oder mehr, beispielsweise 240° oder mehr. Für eine einfache Montage einer Elektrode in einem Schaft mit einem fest verbauten Isoliereinsatz kann es jedoch vorteilhaft sein, wenn der Grad der Umschließung gering gehalten wird. Ein Teil der Innenwand des Isoliereinsatzes, der den jeweiligen Tragarm ebenfalls angrenzend umschließt, wird als Teil des Führungselements angesehen. Grenzt somit die Innenwand des Isoliereinsatzes auf 20° an den Außenumfang des Tragarmes an und eine aus der Innenwand hinausragende Extrusion an weitere 120° des Tragarmes, so umschließt das aus einem Teil der Innenwand und der Extrusion gebildete Führungselement den Tragarm auf 140°.

In einer Ausführungsform ist es angedacht, dass das Führungselement den ihm zugeordneten Tragarm nicht vollständig umschließt. Auf diese Weise kann der Tragarm bei Bedarf seitlich aus dem Führungselement herausgeführt werden und zur Montage in das Führungselement auch seitlich eingeführt werden. Dazu kann das Führungselement eine Flexibilität aufweisen, die ausreichend ist, um durch leichtes Aufbiegen des Elements den Tragarm aus dem Element herauszuführen. In dieser Ausführungsform umschließt das Führungselement den Tragarm auf 90° bis 240° seines Umfangs, vorzugsweise auf 180° bis 240°. Diese Ausführungsform ist besonders für die Montage des Elektrodeninstruments in einem Resektoskop geeignet, das einen fest mit dem Resektoskopschaft verbundenen Isoliereinsatz aufweist. Das Elektrodeninstrument kann für die einmalige Verwendung ausgebildet sein, während der Rest des Resektoskops inklusive des Isoliereinsatzes für die mehrmalige Verwendung und Aufbereitung (Reinigung) ausgebildet sein können.

In einer alternativen Ausführungsform ist das Führungselement derart von dem Tragarm umschlossen, dass der Tragarm nicht ohne Beschädigung des Führungselements aus dem Führungselement entfernt werden kann. Dies kann beispielsweise sinnvoll sein, wenn der Isoliereinsatz Teil des für den einmaligen Einsatz ausgebildeten Elektrodensystems sein soll. Der Isoliereinsatz ist in dieser Ausführungsform entsprechend ebenfalls für die einmalige Verwendung ausgebildet. In dieser und anderen Ausführungsformen umschließt das Führungselement den jeweiligen Tragarm auf 180° oder mehr seines Umfangs, vorzugsweise auf 240° oder mehr, beispielsweise auf 360°, d.h. vollständig.

Entsprechend betrifft die Erfindung in einem verwandten Aspekt auch ein, vorzugsweise zur einmaligen Verwendung ausgebildetes, Elektrodensystem zur Verwendung in einem Resektoskop für die endoskopische Chirurgie, das einen hierin beschriebenen Isoliereinsatz und ein hierin beschriebenes Elektrodeninstrument umfasst. Spezifisch betrifft dieser Aspekt ein Elektrodensystem, das dadurch gekennzeichnet ist, dass es ein Elektrodeninstrument und einen elektrisch isolierenden Isoliereinsatz umfasst, wobei das Elektrodeninstrument einen langgestreckten Schaftabschnitt mit einem oder zwei Tragarmen und in seinem distalen Endbereich eine mit Hochfrequenzstrom beaufschlagbare Elektrode aufweist, und wobei der Isoliereinsatz ein oder mehrere Führungselemente zur Halterung jeweils eines Tragarms des Elektrodeninstruments aufweist, wobei der jeweilige Tragarm in dem Führungselement längsverschiebbar angeordnet ist.

Das Elektrodensystem kann in einem erfindungsgemäßen Resektoskop verwendet werden, aber separat von dem Resektoskop vertrieben werden. Das Elektrodensystem kann daher beispielsweise für die einmalige Verwendung ausgebildet sein.

Das oder die Führungselemente des Elektrodensystems umschließen den jeweiligen Tragarm auf 180° oder mehr seines Umfangs, vorzugsweise 240° oder mehr, am stärksten bevorzugt auf 360°.

### Kurze Beschreibung der Figuren

In den Zeichnungen sind Ausführungsbeispiele der Erfindung schematisch dargestellt. Es zeigen:
- **Fig. 1**: eine seitliche, schematische Schnittdarstellung eines Resektoskops aus dem Stand der Technik, das ein Elektrodeninstrument mit Führungselementen umfasst, die an dem Elektrodeninstrument angebracht sind;
- **Fig. 2**: eine seitliche, schematische Schnittdarstellung eines erfindungsgemäßen Resektoskops, das einen Isoliereinsatz mit Führungselementen für die Tragarme eines Elektrodeninstruments aufweist;
- **Fig. 3**: eine seitliche, schematische Schnittdarstellung des distalen Endbereichs eines Resektoskops aus dem Stand der Technik, das ein Elektrodeninstrument mit Führungselementen umfasst, die an dem Elektrodeninstrument angebracht sind und die sich an der Optik des Systems abstützen;
- **Fig. 4**: eine seitliche, schematische Schnittdarstellung des distalen Endbereichs eines erfindungsgemäßen Resektoskops, das einen Isoliereinsatz mit Führungselementen (gestrichelt dargestellt) für die Tragarme eines Elektrodeninstruments aufweist;
- **Fig. 5**: eine schematische Frontalansicht des Schaftes eines erfindungsgemäßen Resektoskops, das einen Isoliereinsatz mit Führungselementen für die Tragarme eines Elektrodeninstruments aufweist;
- **Fig. 6**: eine schematische Frontalansicht des Schaftes eines alternativen erfindungsgemäßen Resektoskops, das einen Isoliereinsatz mit Führungselementen für die Tragarme eines Elektrodeninstruments aufweist und in dem die Tragarme zwischen Innen- und Hüllrohr verlaufen und in dem die Führungselemente die Tragarme nicht vollständig umschließen;
- **Fig. 7**: eine schematische Frontalansicht des Schaftes eines alternativen erfindungsgemäßen Resektoskops, das einen Isoliereinsatz mit Führungselementen für die Tragarme eines Elektrodeninstruments aufweist und in dem die Tragarme zwischen Innen- und Hüllrohr verlaufen und in dem die Führungselemente die Tragarme nicht vollständig umschließen; und
- **Fig. 8**: eine seitliche, schematische Schnittdarstellung des distalen Endbereichs eines erfindungsgemäßen Elektrodensystems, das einen Isoliereinsatz mit Führungselementen (gestrichelt dargestellt) für die Tragarme eines Elektrodeninstruments sowie ein entsprechendes Elektrodeninstrument umfasst.

### Ausführungsbeispiele

Weitere Vorteile, Kennzeichen und Merkmale der vorliegenden Erfindung werden bei der nachfolgenden detaillierten Beschreibung von Ausführungsbeispielen anhand der beigefügten Zeichnungen deutlich. Allerdings ist die Erfindung nicht auf diese Ausführungsbeispiele beschränkt.

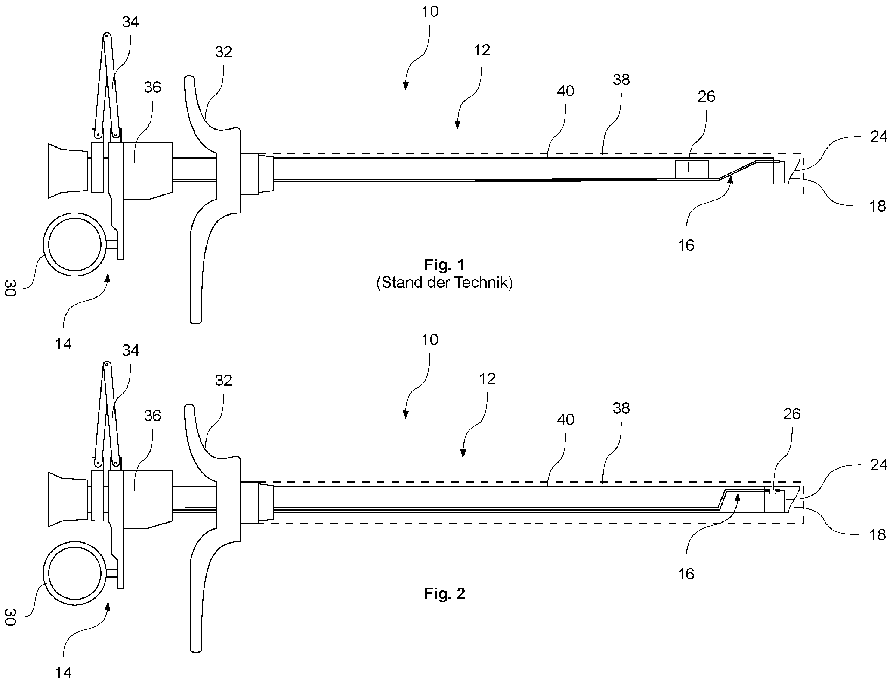

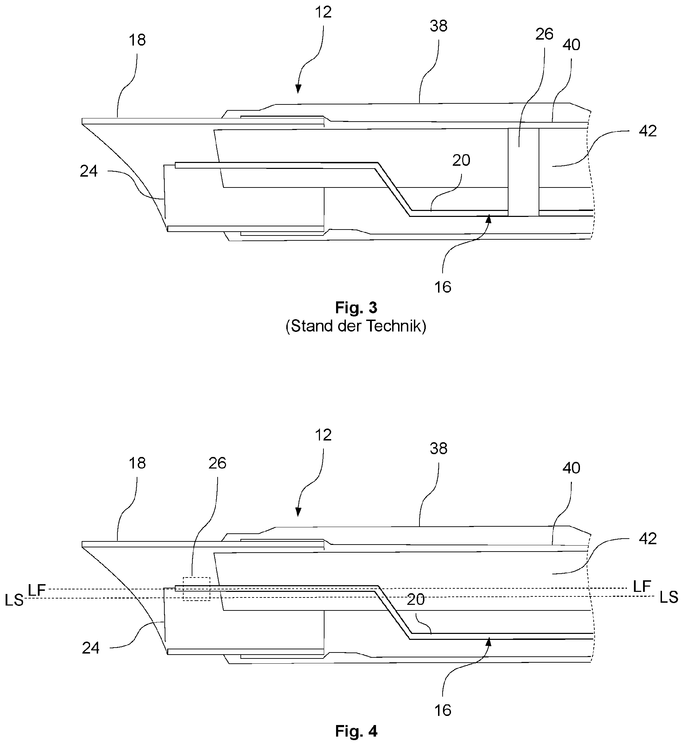

Fig. 1 zeigt eine schematische, seitliche Schnittdarstellung eines Resektoskops 10 aus dem Stand der Technik in dem am Innenrohr 40 ein Isoliereinsatz 18 angeordnet ist und an dessen Elektrodeninstrument 16 Führungselemente 26 angeordnet sind, die sich an einer nicht dargestellten Optik im Inneren des Innenrohres 40 abstützen. Fig. 3 zeigt eine schematische, seitliche Schnittdarstellung des distalen Endbereichs desselben Resektoskops 10 aus dem Stand der Technik.

Das Resektoskop 10 weist einen Schaft 12 auf, der ein gestrichelt dargestelltes Hüllrohr 38 (Außenrohr) umfasst. Innerhalb des Hüllrohres 38 verläuft ein Innenrohr 40 und innerhalb des Innenrohres 40 ein Elektrodeninstrument 16 sowie eine in Fig. 3 dargestellte Optik 42 und ein nicht dargestelltes Beleuchtungsmittel, zum Beispiel in Form eines Lichtleitfaserbündels. Darüber hinaus können weitere hier nicht dargestellte Elemente in den Resektoskopen verlaufen, wie beispielsweise ein separates Spülrohr und dergleichen. Das Hüllrohr 38 umfasst in seinem distalen Endbereich in hier nicht dargestellter Weise Öffnungen durch die verunreinigte Spülflüssigkeit in den Zwischenraum zwischen Hüllrohr 38 und Innenrohr 40 einströmen und durch den Resektoskopschaft 12 abfließen kann.

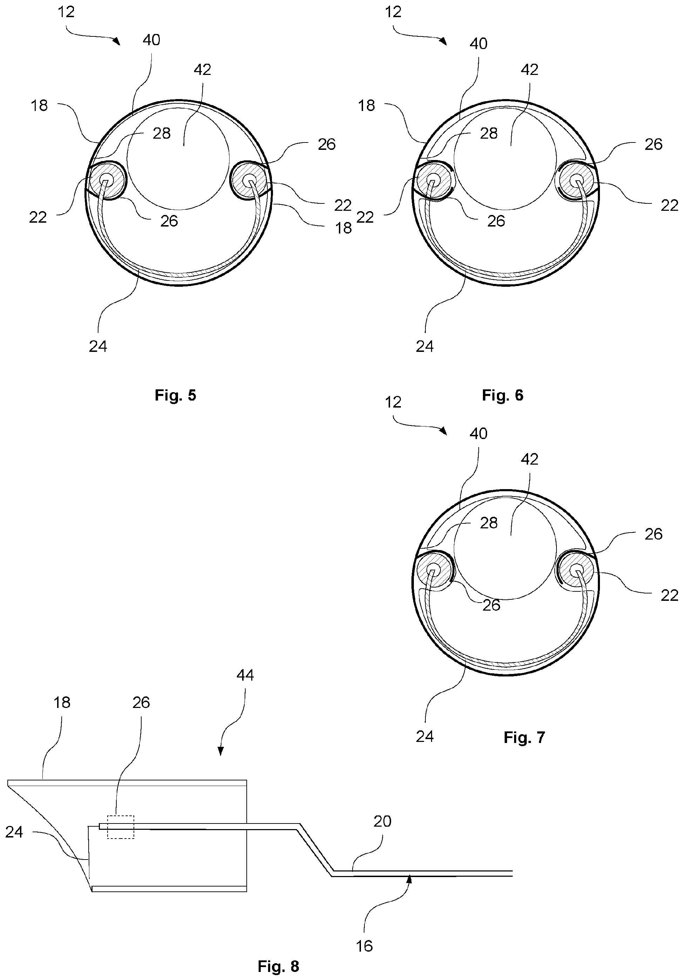

Wie in Fig. 1 und in größerem Detail in der Fig. 3 erkennbar ist, soll ein Elektrodeninstrument 16 in diesem üblichen Instrument mittels eines Führungselements 26 mit einem teilkreisförmigen Querschnitt gegen Verschiebungen abweichend von der Längsrichtung des Schaftes 12, zum Beispiel quer zur Längsrichtung, geschützt sein. Das Elektrodeninstrument 16 ist längsverschiebbar in dem Innenrohr 40 gelagert. Das Führungselement 26 ist formkomplementär zur Außenwand der Optik 42 ausgebildet und weist eine teilzylindrische Form auf. Das Führungselement 26 ist in einem Schaftabschnitt 20 des Elektrodeninstruments 16 an zwei Tragarmen (Gabelrohren) 22 (Fig. 5) befestigt. Die Tragarme 22 verlaufen innerhalb des Resektoskopschaftes 12 dicht nebeneinander und laufen erst im distalen Endbereich des Resektoskopschaftes 12 auseinander, um zwischen ihren Enden eine Elektrode 24 in Form einer Schlingenelektrode aufzunehmen und zu tragen. Die Führungselemente 26 des Elektrodeninstruments 16 helfen nicht gegen eine Verbiegung des Elektrodeninstruments 16 in dem Bereich, der sich distal von den Führungselementen 26 befindet.

Das Elektrodeninstrument 16 kann durch Betätigung eines Handgriffs 14 zwangsgeführt axial in distale und proximale Richtung bewegt werden. Dabei kann es über das distale Ende des Innenrohres 40 und des Hüllrohres 38 hinausgeschoben werden. So wird es dem Operateur ermöglicht, auch weiter von der Resektoskopspitze entferntes Gewebe zu manipulieren. Zu diesem Zweck sind ferner Innenrohr 40 und/oder das Elektrodeninstrument 16 um ihre Längsachsen drehbar gelagert. Das Elektrodeninstrument 16 weist an seinem distalen Ende eine Elektrode 24 auf, die als Schneidschlinge ausgebildet ist und mittels derer Gewebe durch elektrochirurgische Ablation entfernt werden kann. Hierbei wird an die Elektrode 24 eine hochfrequente, elektrische Spannung angelegt, um Gewebe zu zerschneiden.

Das dargestellte Resektoskop 10 weist einen passiven Transporteur auf, bei dem der Schlitten 36 durch Relativbewegung der proximal von dem Resektoskopschaft 12 angeordneten Griffteile 30 und 32 zueinander gegen eine von einer Federbrücke 34 aufgebrachte Federkraft in distale Richtung gegen das distale, erste Griffteil 32 verschoben wird. Bei der Verschiebung des Schlittens 36 in distale Richtung gegen das Griffteil 32 wird das Elektrodeninstrument 16 in nicht dargestellter Weise zwangsgeführt nach distal verschoben. Bei einer Entlastung der Griffteile 30, 32 zwingt die von der Federbrücke 34 erzeugte Federkraft den Schlitten 36 zurück in seine Ruhrposition, wobei das Elektrodeninstrument 16 in proximale Richtung gezogen wird. Bei der Rückverschiebung des Schlittens 36 kann ohne Handkraft des Operateurs, also passiv, ein elektrochirurgischer Eingriff mit dem Elektrodeninstrument 16 vorgenommen werden.

Der Isoliereinsatz 18 des Resektoskops 10 ist an dessen distalen Ende nicht lösbar durch eine Verklebung mit diesem verbunden. Der Isoliereinsatz 18 enthält keine Führungselemente. Er ist aus einer elektrisch isolierenden Keramik ausgebildet.

Fig. 2 und Fig. 4 zeigen schematische, seitliche Schnittdarstellungen eines erfindungsgemäßen Resektoskops 10. Das erfindungsgemäße Resektoskop 10 unterscheidet sich von dem in den Fig. 1 und Fig. 3 gezeigten dadurch, dass die Führungselemente 26 zur Stabilisierung der Längsbewegung des Elektrodeninstruments 16 nicht an dem Schaftabschnitt 20 des Elektrodeninstruments 16 angeordnet sind, sondern an der Innenwand 28 des Isoliereinsatzes 18. Durch die Anbringung der Führungselemente 26 im Inneren des Isoliereinsatzes 18 ist der maximale Abstand zwischen den Führungselementen 26 und der Elektrode 24 auf ein Minimum reduziert. Die Gefahr einer Verbiegung des distalen Endbereichs des Elektrodeninstruments 16 ist hierdurch deutlich verringert, auch wenn das Elektrodeninstrument 16 maximal in distale Richtung verschoben ist. Die Führungselemente 26 sind etwa 0,5 cm von dem distalen Ende des Isoliereinsatzes 18 beabstandet. Die Längsachse des Führungselements (LF) ist dabei in etwa parallel zu der Längsachse des Schaftteils (LS). In Fig. 4 ist erkennbar, dass die Führungselemente 26 in Längsrichtung des Schaftes 12 länglich ausgebildet sind.

In Fig. 5, die Teile desselben Resektoskops 10 zeigt wie Fig. 2 und Fig. 4, nämlich eine schematische Frontalansicht des Schaftes 12, ist erkennbar, dass das Elektrodeninstrument 16 zwei Tragarme 22 aufweist, zwischen deren distalen Enden eine Elektrode 24 gehalten wird, welche die Form einer Schlingenelektrode aufweist. Die Tragarme 22 werden in ihrem Inneren von einem leitfähigen Draht durchlaufen und umfassen darüber hinaus eine den Draht ummantelnde Isolierschicht, welche die Tragarme 22 nach außen elektrisch isoliert. Das Innenrohr 40 weist darüber hinaus eine Optik 42 auf, die zwischen den Tragarmen 22 verläuft.

Es ist ferner erkennbar, dass der Isoliereinsatz 18, der an dem distalen Ende des Resektoskopschaftes 12 angeordnet ist, zwei Führungselemente 26 aufweist. Die Führungselemente 26 liegen einander - aus der Sagittalebene des Isoliereinsatzes 18 betrachtet - gegenüber. Jeder der zwei Tragarme 22 des Elektrodeninstruments 16 verläuft durch ein Führungselement 26 des Isoliereinsatzes 18. Die Führungselemente 26 sind aus Gründen der Platzersparnis mindestens teilweise formkomplementär zu den Tragarmen 22. Die Führungselemente 26 weisen jeweils eine Teilkreisform auf, deren Enden mit der Innenwand 28 des Isoliereinsatzes 18 verbunden sind. In der gezeigten Ausführungsform sind die Führungselemente 26 einteilig mit dem Isoliereinsatz 18 aus einer elektrisch isolierenden Keramik ausgebildet. Die Führungselemente 26 umschließen den jeweiligen Tragarm 22 vollständig, d.h. auf 360° seines Umfangs.

Fig. 6 zeigt eine schematische Frontalansicht des Schaftes 12 eines alternativen erfindungsgemäßen Resektoskops 10, das einen Isoliereinsatz 18 mit Führungselementen 26 für die Tragarme 22 eines Elektrodeninstruments 16 aufweist. Das Resektoskop 10 unterscheidet sich von dem in Fig. 5 dargestellten dadurch, dass die Tragarme 22 des Elektrodeninstruments 16 nicht innerhalb des Innenrohres 40 sondern zwischen dem Innenrohr 40 und dem Hüllrohr 38 angeordnet sind. Darüber hinaus umschließen die Führungselemente 26 die Tragarme 22 im Gegensatz zu den in Fig. 5 dargestellten nicht vollständig. Die Führungselemente 26 weisen jeweils eine Unterbrechung auf, die zur seitlichen Einführung der Tragarme 22 genutzt werden kann. Dazu sind die Führungselemente 26 ausreichend flexibel, um eine Vergrößerung der Unterbrechung während der Montage oder Demontage zu erlauben, ohne zu verbiegen oder zu brechen. Die Unterbrechung in den Führungselementen 26 ist in der gezeigten Ausführungsform in Richtung der Optik bzw. in Richtung des zweiten Tragarms 22 ausgebildet. Die Unterbrechungen der beiden Führungselemente 26 liegen einander somit gegenüber. Die Unterbrechungen erstrecken sich in hier nicht dargestellter Weise in Längsrichtung des Instruments über die gesamte Länge der Führungselemente 26.

Fig. 7 zeigt eine zu Fig. 6 alternative Ausbildungsweise für Tragarme 22, die nicht zur vollständigen Umschließung der Führungselemente 26 ausgebildet sind. Die Führungselemente 26 weisen auch hier jeweils eine Unterbrechung auf. Die Unterbrechung ist in Richtung einer Transversalebene des Resektoskopschaftes 12 ausgebildet. Die Unterbrechungen erstrecken sich in hier nicht dargestellter Weise in Längsrichtung des Instruments über die gesamte Länge der Führungselemente 26.

Fig. 8 zeigt eine seitliche, schematische Schnittdarstellung des distalen Endbereichs eines erfindungsgemäßen Elektrodensystems 44, das einen Isoliereinsatz 18 mit Führungselementen 26 (gestrichelt dargestellt) für die Tragarme 22 eines Elektrodeninstruments 16 sowie ein entsprechendes Elektrodeninstrument 16 umfasst. Das Elektrodensystem 44 ist für die einmalige Verwendung ausgelegt und kann nach Verwendung während eines medizinischen Eingriffs entsorgt werden. Alternativ ist auch ein zu einer mehrmaligen Verwendung ausgelegtes Elektrodensystem 44 denkbar. Der Isoliereinsatz 18 in dem Elektrodensystem 44 erleichtert die Montage des Elektrodensystems an dem Resektoskopschaft 12, da der Isoliereinsatz 18 im Gegensatz zu dem empfindlichen Elektrodeninstrument 16 während der Montage ohne Gefahr eines Verbiegens angefasst werden kann. Der Isoliereinsatz 18 kann zur Befestigung hier nicht dargestellte Rastmechanismen oder andere Befestigungsmittel umfassen, wie beispielsweise die in der DE 10 2019 102 841.8 beschriebenen Befestigungsmittel. Die Führungselemente 26 sind in diesem Falle zur vollständigen Umschließung des jeweiligen Tragarmes 22 ausgebildet.

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird. Die vorliegende Offenbarung schließt sämtliche Kombinationen der vorgestellten Einzelmerkmale ein.

## Patentansprüche

1. Elektrodensystem (44) zur Verwendung in einem Resektoskop (10) für die endoskopische Chirurgie, wobei das Elektrodensystem ein Elektrodeninstrument (16) und einen elektrisch isolierenden Isoliereinsatz (18) umfasst,
wobei das Elektrodeninstrument (16) einen langgestreckten Schaftabschnitt (20) mit einem oder zwei Tragarmen (22) und in seinem distalen Endbereich eine mit Hochfrequenzstrom beaufschlagbare Elektrode (24) aufweist, **dadurch gekennzeichnet, dass** der Isoliereinsatz (18) ein oder mehrere Führungselemente (26) zur Halterung jeweils eines Tragarms (22) des Elektrodeninstruments (16) aufweist, wobei der Tragarm (22) in dem Führungselement (26) längsverschiebbar angeordnet ist.

2. Elektrodensystem (44) nach Anspruch 1, **dadurch gekennzeichnet, dass** das oder die Führungselemente (26) den jeweiligen Tragarm (22) auf 240° oder mehr seines Umfangs umschließen, vorzugsweise auf 360°.

3. Resektoskop (10) für die endoskopische Chirurgie mit einem rohrartigen Schaft (12) und einem Handgriff (14), wobei das Resektoskop (10) das Elektrodensystem (44) gemäß Anspruch 1 oder 2 umfasst, und wobei das Elektrodeninstrument (16) im rohrartigen Schaft (12) längsverschieblich angeordnet ist und an seinem distalen Ende einen elektrisch isolierenden Isoliereinsatz (18) umfasst.

4. Resektoskop (10) nach Anspruch 3, **dadurch gekennzeichnet, dass** das Elektrodeninstrument (16) zwei Tragarme (22) aufweist.

5. Resektoskop (10) nach einem der Ansprüche 3 oder 4, **dadurch gekennzeichnet, dass** der Abstand zwischen dem distalen Ende des Schafts (12) und dem oder den Führungselementen (26) 1 cm oder weniger beträgt.

6. Resektoskop (10) nach einem der vorangegangenen Ansprüche 3 bis 5, **dadurch gekennzeichnet, dass** das oder die Führungselemente (26) an der Innenwand (28) des Isoliereinsatzes (18) angeordnet sind.

7. Resektoskop (10) nach einem der vorangegangenen Ansprüche 3 bis 6, **dadurch gekennzeichnet, dass** die Längsachsen (LF) des oder der Führungselemente (26) jeweils parallel zur Längsachse (LS) des Schaftes (12) verlaufen.

8. Resektoskop (10) nach einem der vorangegangenen Ansprüche 3 bis 7, **dadurch gekennzeichnet, dass** das oder die Führungselemente (26) den jeweiligen Tragarm (22) auf 90° oder mehr seines Umfangs umschließen.

9. Resektoskop (10) nach einem der vorangegangenen Ansprüche 3 bis 8, **dadurch gekennzeichnet, dass** das oder die Führungselemente (26) den jeweiligen Tragarm (22) auf 240° oder mehr seines Umfangs umschließen, vorzugsweise auf 360°.

10. Resektoskop (10) nach einem der vorangegangenen Ansprüche 3 bis 9, **dadurch gekennzeichnet, dass** die Elektrode (24) eine Schlingenelektrode ist.

## Claims

1. An electrode system (44) for use in a resectoscope (10) for endoscopic surgery, wherein the electrode system comprises an electrode instrument (16) and an electrically insulating insert (18), wherein the electrode instrument (16) has an elongated shaft section (20) with one or two support arms (22) and in its distal end region an electrode (24) that can be acted upon with high-frequency current, **characterized in that** the insulating insert (18) has one or more guide elements (26) for holding a respective support arm (22) of the electrode instrument (16), the support arm (22) being arranged in the guide element (26) so as to be longitudinally displaceable.

2. The electrode system (44) as claimed in claim 1, **characterized in that** the guide element or elements (26) surround the respective support arm (22) over 240° or more of its circumference, preferably over 360°.

3. A resectoscope (10) for endoscopic surgery with a tubular shaft (12) and a handle (14), wherein the resectoscope (10) comprises the electrode system (44) pursuant to claim 1 or 2, and wherein the electrode instrument (16) is arranged in the tubular shaft (12) so as to be longitudinally displaceable and, at its distal end, has an electrically insulating insert (18).

4. The resectoscope (10) as claimed in claim 3, **characterized in that** the electrode instrument (16) has two support arms (22).

5. The resectoscope (10) as claimed in any one of claims 3 or 4, **characterized in that** the distance between the distal end of the shaft (12) and the guide element or elements (26) is 1 cm or less.

6. The resectoscope (10) as claimed in any one of the preceding claims 3 to 5, **characterized in that** the guide element or elements (26) are arranged on the inner wall (28) of the insulating insert (18).

7. The resectoscope (10) as claimed in any one of the preceding claims 3 to 6, **characterized in that** the longitudinal axes (LF) of the guide element or elements (26) in each case run parallel to the longitudinal axis (LS) of the shaft (12).

8. The resectoscope (10) as claimed in any one of the preceding claims 3 to 7, **characterized in that** the guide element or elements (26) enclose the respective support arm (22) over 90° or more of its circumference.

9. The resectoscope (10) as claimed in any one of the preceding claims 3 to 8, **characterized in that** the guide element or elements (26) enclose the respective support arm (22) over 240° or more of its circumference, preferably over 360°.

10. The resectoscope (10) as claimed in any one of the preceding claims 3 to 9, **characterized in that** the electrode (24) is a loop electrode.

## Revendications

1. Système d'électrode (44) destiné à être utilisé dans un résectoscope (10) pour la chirurgie endoscopique, le système d'électrode comprenant un instrument d'électrode (16) et un insert électriquement isolant (18), l'instrument d'électrode (16) comportant une partie de tige allongée (20) dotée d'un ou deux bras de support (22) et, dans sa zone d'extrémité distale, d'une électrode (24) pouvant être alimentée en courant à haute fréquence, **caractérisé en ce que** l'insert isolant (18) comporte un ou plusieurs éléments de guidage (26) destinés à maintenir respectivement chaque bras de support (22) de l'instrument d'électrode (16), le bras de support (22) étant agencé de manière à pouvoir coulisser longitudinalement dans l'élément de guidage (26).

2. Système d'électrode (44) selon la revendication 1, **caractérisé en ce que** le ou les éléments de guidage (26) entourent le bras de support (22) respectif sur 240° ou plus de sa circonférence, de préférence sur 360°.

3. Résectoscope (10) pour la chirurgie endoscopique, doté d'une tige tubulaire (12) et d'une poignée (14), le résectoscope (10) comprenant le système d'électrode (44) selon la revendication 1 ou 2, et l'instrument d'électrode (16) étant agencé de manière à pouvoir coulisser longitudinalement dans la tige tubulaire (12) et comprenant à son extrémité distale un insert électriquement isolant (18).

4. Résectoscope (10) selon la revendication 3, **caractérisé en ce que** l'instrument d'électrode (16) comporte deux bras de support (22).

5. Résectoscope (10) selon la revendication 3 ou 4, **caractérisé en ce que** la distance entre l'extrémité distale de la tige (12) et le ou les éléments de guidage (26) est de 1 cm ou moins.

6. Résectoscope (10) selon l'une quelconque des revendications précédentes 3 à 5, **caractérisé en ce que** le ou les éléments de guidage (26) sont agencés sur la paroi intérieure (28) de l'insert isolant (18).

7. Résectoscope (10) selon l'une quelconque des revendications 3 à 6 précédentes, **caractérisé en ce que** les axes longitudinaux (LF) du ou des éléments de guidage (26) sont respectivement parallèles à l'axe longitudinal (LS) de la tige (12).

8. Résectoscope (10) selon l'une quelconque des revendications 3 à 7 précédentes, **caractérisé en ce que** le ou les éléments de guidage (26) entourent le bras de support (22) respectif sur 90° ou plus de sa circonférence.

9. Résectoscope (10) selon l'une quelconque des revendications 3 à 8 précédentes, **caractérisé en ce que** le ou les éléments de guidage (26) entourent le bras de support (22) respectif sur 240° ou plus de sa circonférence, de préférence sur 360°.

10. Résectoscope (10) selon l'une quelconque des revendications 3 à 9 précédentes, **caractérisé en ce que** l'électrode (24) est une électrode à boucle.
